# EUROPÄISCHE PATENTANMELDUNG

(11) **EP 1 034 742 A1**
(43) Veröffentlichungstag der Anmeldung: **13.09.2000**
(21) Anmeldenummer: 99890078.1
(22) Anmeldetag: 09.03.1999
(51) Int. Cl.: A61B 8/00, G01N 29/00, G01S 15/00

(54) **Verfahren zur Untersuchung von Objekten mit Ultraschall**

(71) Anmelder: Kreztechnik Aktiengesellschaft, 4871 Zipf (AT)
(72) Erfinder: Wiesauer, Franz, Dipl. Ing., 4871 Zipf (AT); Gritzky, Arthur, Dipl. Ing., 4710 Pollham (AT)
(74) Vertreter: Hübscher, Heiner, Dipl.-Ing.

(57) **Zusammenfassung**

Bei einem Verfahren zur Untersuchung von Objekten mit Ultraschall wird ein Volumsbereich des Objektes mittels einer 3D-Ultraschallsonde mit Ultraschallwellen abgetastet. Aus den von den Echoimpulsen erhaltenen Signalen werden nach wählbaren Abfragekriterien nur die diese Kriterien erfüllenden Signale ausgewählt und an dem Ort ihrer Entstehung geometrisch zugeordneter Stelle zum Aufbau von Bilddarstellungen auf wenigstens einer Anzeigeeinheit verwendet. Für eine Bilddarstellung aus einem mehrschichtigen oder unterschiedliche Einzelkörper umfassenden Volumsbereich werden als geometrisches Auswahlkriterium zumindest näherungsweise eine eine Schicht - oder Körperoberfläche begrenzende Schale bestimmt und nur die dieses Kriterium erfüllenden Signale zur Bilddarstellung verwendet, so daß der Körper in der Darstellung besser hervortritt und vor allem Informationen über die Oberflächenbeschaffenheit oder Durchblutung darstellbar sind.

## Beschreibung

Die Erfindung betrifft ein Verfahren zur Untersuchung von Objekten mit Ultraschall, bei dem ein Volumsbereich des Objektes mittels einer 3D-Ultraschallsonde mit Ultraschallwellen abgetastet und aus den von den Echoimpulsen erhaltenen Signalen nach wählbaren Abfragekriterien nur die diese Kriterien erfüllenden Signale ausgewählt und an dem Ort ihrer Entstehung geometrisch zugeordneter Stelle zum Aufbau von Bilddarstellungen auf wenigstens einer Anzeigeeinheit verwendet werden.

Die Abtastung eines Volumenbereiches eines zu untersuchenden Objektes bei lagerichtiger Speicherung der aus dieser Abtastung erhaltenen Signale ist bekannt (AT 358 155 B). Dabei wird eine Abtastebene (B- oder C-Bild) über das zu untersuchende Objekt geschwenkt. Dieser Schwenk wird entweder manuell durchgeführt (bei gleichzeitiger Messung der Position dere Abtastebene bezüglich einer Referenzposition) oder durch spezielle Schallsonden, die diese Schwenkbewegung automatisch durchführen (z. B. AT 1708 GM). Die Volumsabtastung wird sowohl mit einem über eine Schnittebene mechanisch verstellten und zusätzlich normal dazu verschwenkten Schallkopf als auch mit quer zur Abtastebene schwenkenden Multielementschallköpfen durchgeführt. An sich ist es bei der Volumsabtastung bisher möglich, aus den lagerichtig gespeicherten Echoinformationen das Ultraschallbild beliebiger Schnittebenen zu rekonstruieren, wobei diese Schnittebenen nichts mehr mit einer der ursprünglichen Abtastebenen zu tun haben, die zum Aufbau des Volumendatensatzes verwendet werden. Es können also Bilder erzeugt werden, die aus anatomischen Gründen etwa für eine B-Bildabtastung nicht zugänglich sind. Durch Visualisierungstechniken können Echos, die von einer reflektierenden Oberfläche im Objekt kommen, so dargestellt werden, daß beim Betrachter ein 3D-Eindruck entsteht. Der Blickwinkel, aus dem das Objekt rekonstruiert wird, ist unabhängig von der Aufnahmerichtung und man kann mit dieser Technik "virtuell" um das Objekt herumgehen, um die entsprechenden Bildeindrücke auf einem Monitor sichtbar zu machen. Bereits bei der üblichen 3D-Darstellung ist ein kritischer Punkt der Visualisierung die Entfernung störender Informationen, die den Blick auf das gewünschte Objekt verdecken. Ein typisches Szenario in der Geburtshilfe ist die Darstellung des Fötus durch abdominalen Ultraschall. Dabei werden zwischen der Ultraschallsonde und dem Fötus Echos vom Bauchgewebe der Mutter dargestellt, die den Blick auf das Gesicht oder den Körper des Föten verdecken. Bisher wird einfach aus dem abgetasteten Volumsbereich ein "Volumen von Interesse" ausgewählt und dargestellt, wobei die Begrenzung dieses "Volumens von Interesse" in die Nähe des für die Untersuchung bestimmten Körpers oder der entsprechenden Gewebeschicht herangelegt wird, aber immer noch störende Informationen oder uninteressante Bereiche umfaßt. Überdies können bei den beschriebenen Darstellungsarten kaum weitere Informationen über die tatsächliche Beschaffenheit der Oberfläche des interessierenden Körpers erhalten werden und die Darstellung bzw. die Auswahl des "Volumens von Interesse" erfordert Sachkenntnisse der Bedienungsperson und ein nicht immer vorhandenes Fingerspitzengefühl. Zusätzlich zur Beschaffenheit der Oberfläche wären in vielen Fällen auch weitere Informationen zur Erzielung eines vollständigen Bildes über das untersuchte Objekt von Interesse. Dazu zählen u. a. Informationen über kleinste Relativbewegungen in der Oberfläche des untersuchten Objektes und vor allem Informationen über die vorhandene Durchblutung oder die Durchblutungsverteilung des untersuchten Objektbereiches, da z. B. bei der Nierenuntersuchung aus der Durchblutungsverteilung auf krankhafte Veränderungen geschlossen werden kann.

Aufgabe der Erfindung ist demnach eine Verbesserung des bekannten Verfahrens mit dem Ziel, die Darstellung eines Körpers oder einer Schicht im "Volumen von Interesse" hinsichtlich der Bedienungskriterien für das verwendete Gerät wesentlich zu vereinfachen und überdies aus der Ultraschalluntersuchung die Ableitung von Informationen über Oberflächenbeschaffenheit, Durchblutung usw. des "Objektes von Interesse" zu ermöglichen.

Die gestellte Aufgabe wird bei einem Verfahren der eingangs genannten Art dadurch gelöst, daß für eine Bilddarstellung aus einem mehrschichtigen oder unterschiedliche Einzelkörper umfassenden Volumsbereich als geometrisches Auswahlkriterium zumindest näherungsweise eine eine Schicht- oder Körperoberfläche begrenzende Schale bestimmt und nur die dieses Kriterium erfüllenden Signale zur Bilddarstellung verwendet werden.

Die Einstellung auf die jeweilige Oberfläche kann, falls ihre Reflexionseigenschaften eindeutig definiert oder definierbar sind, automatisch erfolgen. Es ist aber auch eine halbautomatische Einstellung mit Hilfsoperationen, z. B. manueller Einstellung einer im Bild dargestellten Anzeige auf die Oberfläche möglich, wobei bei der 3D-Darstellung die Einstellung auf mehrere Stellen des von mehreren Seiten betrachteten "Objektes von Interesse" erfolgen kann, so daß ein Netzraster bestimmt wird, der das "Objekt von Interesse" umschließt, wobei zwischen Netzlinien flächig interpoliert wird, um die "Schale" zu erhalten. Auch bei der automatischen Einstellung wird man vorzugsweise die Bestimmung der auszuwählenden Oberfläche nach einem aus mehreren Schnittebenenabtastungen erhaltenen Raster vornehmen, wobei die Oberfläche ähnlich wie bei computerunterstützten Konstruktionen aus diesem Raster durch eine CAD-Software rekonstruiert wird. Es ist möglich, in der Darstellung die außerhalb der Schale vorhandenen Darstellungen zu eliminieren, so daß das zu untersuchende Objekt für den Betrachter wesentlich deutlicher als nach der bisherigen Darstellungsart hervortritt. Der Hauptzweck der vorliegenden Erfindung besteht aber darin, aus der "Oberfläche von Interesse" zusätzliche im üblichen 3D-Bild bisher nicht darstellbare Informationen zu erhalten, die die Oberflächenbeschaffenheit bzw. Durchblutung betreffen können.

Nach einer Weiterbildung der Erfindung wird die in die Schicht- bzw. Körperoberfläche hineinreichende Dicke der das Auswahlkriterium darstellenden Schale zwischen annähernd Null und einem größeren, im extrem die gesamte Schicht bzw. den gesamten Körper erfassenden Wert variiert. Hier kann man neben reinen Oberflächeninformationen auch weitere gewünschte Informationen aus dem ausgesuchten Einzelkörper erhalten und beispielsweise sogar in den weiteren Darstellungen Schnittbilder nur aus diesem Körper veranschaulichen. Die Auswahl verschiedener Schichtdicken oder die kontinuierliche Änderung der Schichtdicke der Schale kann - etwa bei Doppler-Auswertungen - zusätzliche Informationen die Durchblutung oder sogar die Strömungsrichtung des Blutes in den Gefäßen geben, die somit sehr übersichtlich dargestellt werden können. Eine ergänzende Möglichkeit besteht darin, eine Dicke der Schale vorzugeben und diese Schale (bei konvex gewölbten Körpern unter entsprechender Verkleinerung) in den jeweils untersuchten Körper hineinwandern zu lassen und so schichtenweise zu untersuchen.

Nach einer der vorhandenen Möglichkeiten werden die ausgewählten Signale einer Histogrammauswertung unterzogen und die erhaltenen Grauwerte und bzw. oder abgeleitete Farbwerte entsprechend dargestellt. Nach einer anderen Möglichkeit, die aber mit der letztgenannten kombinierbar ist, werden die ausgewählten Signale einer Doppler-Auswertung unterzogen und zum Aufbau entsprechender Colordarstellungen verwendet.

Die erhaltenen Bilder können nach einer Möglichkeit für sich allein dargestellt werden. Dabei bietet sich für die Erstuntersuchung eine transparente 3D-Darstellung an. Diese Darstellung kann für sich alleine erfolgen. Nach einer Weiterbildung der Erfindung ist es aber auch möglich, die aus dem Histogramm bzw. der Doppler-Auswertung erhaltenen Darstellungen einer 3D-Darstellung des untersuchten Objektes zu überlagern, so daß sie das "Objekt von Interesse" aus dem gesuchten Gesamtvolumen hervorheben und eine Zuordnung der von der "Oberfläche von Interesse" erhaltenen Informationen zum Gesamtvolumen möglich ist.

Für eine Dokumentation und auch zur Erzielung eines Gesamtüberblickes wird nach einer Variante der Erfindung für die Darstellung eine Transformation unter der Annahme einer Abwicklung der Schale auf einer Ebene vorgenommen.

Weitere Einzelheiten und Vorteile des Erfindungsgegenstandes entnimmt man der nachfolgenden Zeichnungsbeschreibung. In der Zeichnung ist der Erfindungsgegenstand beispielsweise veranschaulicht. Es zeigen
- Fig. 1: ein Blockschaltschema der zur Durchführung des erfindungsgemäßen Verfahrens eingesetzten Elemente eines 3D-Ultraschall-Untersuchungsgerätes,
- Fig. 2: eine Schnittdarstellung zur Verdeutlichung der Auswahlschritte des erfindungsgemäßen Verfahrens und
- Fig. 3: in Zuordnung dazu ein Blockschema der Elemente und der eingesetzten Software.

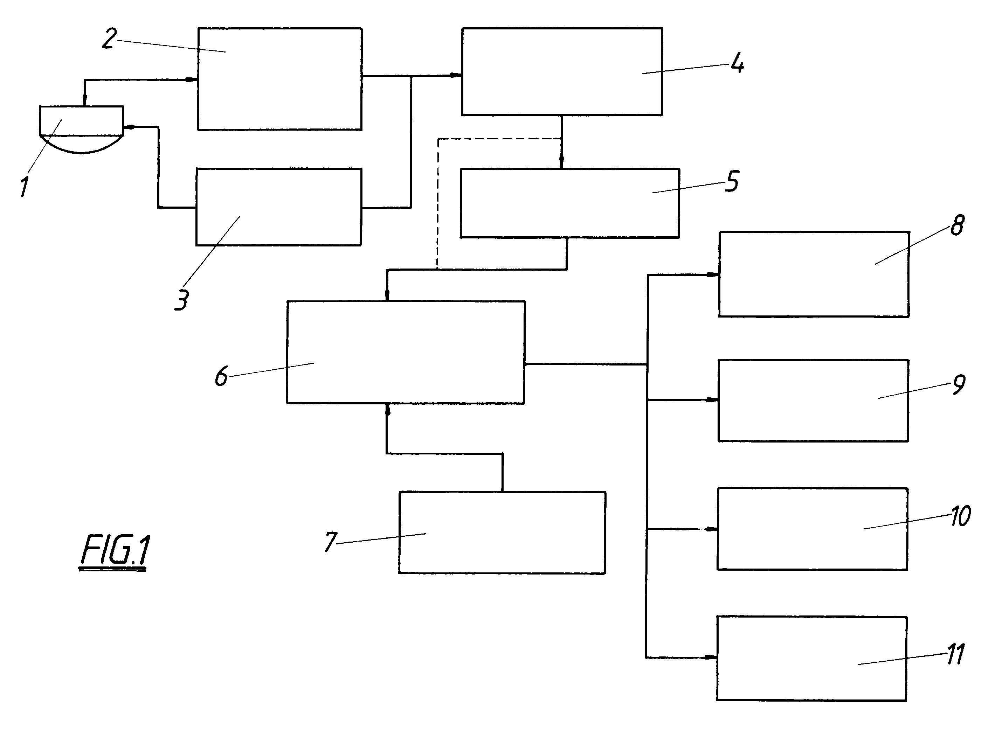

Nach Fig. 1 ist eine 3D-Sonde 1 zur Abtastung eines größeren Volumsbereiches in einem Objekt vorgesehen. Zur Abgabe der Ultraschallimpulse und zum Empfang der zugeordneten Echos dient ein Ultraschall-Echoprozessor 2. Die 3D-Abtastung wird durch eine Steuereinheit 3 bestimmt, die die Stellung der Sonde 1 und die Abgabe der Schallimpulse steuert und überdies einen Speicher 4 ansteuert, wo die erhaltenen Echosignale nach dem Ort ihrer Entstehung gespeichert werden. Eine Echointerpolationsstufe 5 kann im Bedarfsfall vorgesehen werden. Aus dem Speicher 4 werden die Echosignale in einem Koordinatenspeicher 6 überschrieben, der beim Ausführungsbeispiel die Echosignale in Zuordnung zu dem geometrischen Ort ihrer Entstehung nach dem cartesischen Koordinatensystem speichert. Aus diesem Speicher 6 werden die Signale über eine Abfrageeinrichtung 7 abgefragt, wobei die Weiterleitung in verschiedene Auswertungsstufen 8, 9, 10, 11 erfolgt. Die Auswertungsstufe 8 dient zu einer Darstellung aus dem abgetasteten Volumen in mehreren, auswählbaren Abtastebenen, die Auswertungsstufe 9 zur Volumsdarstellung, die Auswertungsstufe 10 für den Aufbau eines Oberflächennetzmodelles der auswählbaren "Fläche von Interesse" und die Stufe 11 schließlich im Zusammenwirken mit der Stufe 10 für die Verwirklichung der erfindungsgemäßen "Schalendarstellung".

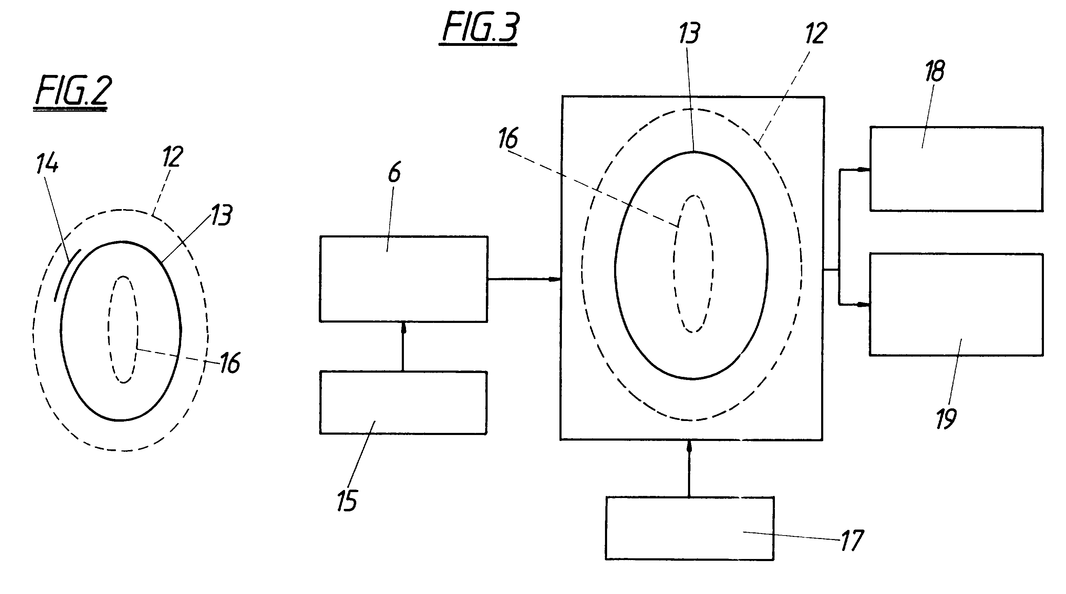

Wie schon in der allgemeinen Beschreibung dargelegt wurde, soll mit der Erfindung aus einem Gesamtvolumen die Oberfläche eines Körpers oder einer besonders interessanten Schicht ausgewählt und dargestellt werden. In Fig.2 wurde strichliert der Umriß 12 eines Gesamtvolumens, das der Abtastung unterzogen wurde, angedeutet. Innerhalb dieses Gesamtvolumens befindet sich ein "Körper von Interesse", dessen Oberfläche mit 13 bezeichnet ist. Die Untersuchung nach dem erfindungsgemäßen Verfahren soll auf diese Oberfläche 13 bezogen werden. Dazu sind verschiedene Auswahlkriterien möglich. Bei einer automatischen Auswahl wird aus von der Reflexion der Oberfläche 13 in mehreren Schnittebenen erhaltenen Oberflächenlinien ein Raster gebildet und ähnlich wie beim CAD durch Interpolation zu der gewünschten Schalenoberfläche ergänzt. Es ist auch möglich, eine der Oberflächenlinie etwa entsprechende Kurve 14 in das von 12 umschlossene Volumen einzublenden und bis zur Dekkung mit 13 zu verstellen, so daß dann die "Schale" definiert ist. Diese Annäherung kann auch automatisch durch eine Schrittsteuerung vorgenommen werden. Ist die Oberfläche des Volumens auf die beschriebene Art definiert, so werden die entsprechenden Adressen der Oberflächenpunkte in einer Steuereinrichtung 15 (siehe ab nun Fig. 3) gespeichert. Zu erwähnen ist noch, daß man wahlweise nur auf die Oberfläche 13 einstellt oder auch von der Oberfläche 13 in die Tiefe gehend verschieden dicke Schichten der definierten Schale bestimmen kann, aus denen dann Signale der Auswertung zugeführt werden. Eine mögliche Innenbegrenzung der Schalenschicht ist in Fig. 2 mit 16 bezeichnet.

Für die weitere Darstellung werden über 15 aus dem Speicher 6 nur die von der "Oberfläche von Interesse" 13 bzw. aus dem darzustellenden Schalenvolumen erhaltenen Signale ausgewählt und der weiteren Verarbeitung zugeführt. Die Art der Signalverarbeitung (Grauwert bzw. Farbdarstellung oder Farbdarstellung einer Dopplerauswertung) wird durch einen Prozessor 17 bestimmt. Softwaremäßig wird über eine Stufe 18 die Gesamtoberfläche des zu untersuchenden Objektes bestimmt und über eine Stufe 19 die Größe des von der Schale umschlossenen Raumes berechnet.

Die möglichen Darstellungsarten wurden in der allgemeinen Beschreibung näher erläutert. Hinsichtlich des Gesamtaufbaues und der Einrichtungen zur 3D-Darstellung wird auf unsere analoge EP 98890169.0 vom 4. Juni 1998 Bezug genommen.

## Patentansprüche

1. Verfahren zur Untersuchung von Objekten mit Ultraschall, bei dem ein Volumsbereich des Objektes mittels einer 3D-Ultraschallsonde mit Ultraschallwellen abgetastet und aus den von den Echoimpulsen erhaltenen Signalen nach wählbaren Abfragekriterien nur die diese Kriterien erfüllenden Signale ausgewählt und an dem Ort ihrer Entstehung geometrisch zugeordneter Stelle zum Aufbau von Bilddarstellungen auf wenigstens einer Anzeigeeinheit verwendet werden, dadurch gekennzeichnet, daß für eine Bilddarstellung aus einem mehrschichtigen oder unterschiedliche Einzelkörper umfassenden Volumsbereich als geometrisches Auswahlkriterium zumindest näherungsweise eine eine Schicht- oder Körperoberfläche begrenzende Schale bestimmt und nur die dieses Kriterium erfüllenden Signale zur Bilddarstellung verwendet werden.

2. Verfahren nach Anspruch 1, dadurch gekennzeichnet, daß die in die Schicht- bzw. Körperoberfläche hineinreichende Dicke der das Auswahlkriterium darstellenden Schale zwischen annähernd Null und einem größeren, im extrem die gesamte Schicht bzw. den gesamten Körper erfassenden Wert variiert wird.

3. Verfahren nach Anspruch 1 oder 2, dadurch gekennzeichnet, daß die ausgewählten Signale einer Histogrammauswertung unterzogen und die erhaltenen Grauwerte und bzw. oder abgeleitete Farbwerte entsprechend dargestellt werden.

4. Verfahren nach Anspruch 1 oder 2, dadurch gekennzeichnet, daß die ausgewählten Signale einer Doppler-Auswertung unterzogen und zum Aufbau entsprechender Colordarstellungen verwendet werden.

5. Verfahren nach einem der Ansprüche 1 bis 4, dadurch gekennzeichnet, daß die aus dem Histogramm bzw. der Doppler-Auswertung erhaltenen Darstellungen einer 3D-Darstellung des untersuchten Objektes überlagert werden.

6. Verfahren nach einem der Ansprüche 1 bis 4, dadurch gekennzeichnet, daß für die Darstellung eine Transformation unter der Annahme einer Abwicklung der Schale auf einer Ebene vorgenommen wird.
